# EUROPEAN PATENT SPECIFICATION

(11) **EP 1 012 154 B1**
(45) Date of publication and mention of the grant of the patent: **17.03.2004**
(21) Application number: 98937115.8
(22) Date of filing: 24.07.1998
(51) Int. Cl.: C07H 1/00

(54) **SALTS OF DI(URIDINE 5'-TETRAPHOSPHATE), METHOD FOR PREPARATION AND USES THEREOF**
SALZE VON DI(URIDINE 5'-TETRAPHOSPHATE), VERFAHREN ZUR HERSTELLUNG UND VERWENDUNGEN DAVON
PROCEDE DE PRODUCTION DE MASSE DE DI(URIDINE 5'-TETRAPHOSPHATE) ET DE SES SELS

(30) Priority: 25.07.1997 US 54147 P
(43) Date of publication of application: 28.06.2000
(73) Proprietor: Inspire Pharmaceuticals, Inc., Durham, NC 27703 (US)
(72) Inventor: YERXA, Benjamin, R., Raleigh, NC 27608 (US); PENDERGAST, William, Durham, NC 27713 (US)
(74) Representative: Schüssler, Andrea, Dr.
(86) International application number: PCT/US1998/015445
(87) International publication number: WO 1999/005155

(56) References cited:
- WO-A-96/40059
- WO-A-97/29756
- WO-A-97/35591
- WO-A-98/03177
- WO-A-98/03182
- WO-A-98/34593
- WO-A-98/34942
- C.G.VALLEJO ET AL.: "Dinucleosidasetetraphosphatase in Rat Liver and Artemia Salina." BIOCHIMICA BIOPHYSICA ACTA, vol. 438, no. 1, 1976, pages 304-309, XP002089062 cited in the application

## Description

This application claims priority to U.S. Provisional Application 60/054,147 filed July 25, 1997.

### TECHNICAL FIELD

This invention relates to methods for the production of therapeutic dinucleotides including novel salts thereof. More specifically, it relates to methods for synthesis of P¹,P⁴-di(uridine 5')-tetraphosphate, i.e., diuridine tetraphosphate (U₂P₄) which have advantages over prior art methods of manufacture.

### BACKGROUND OF THE INVENTION

P¹,P⁴-Di(uridine 5')-tetraphosphate is a dinucleotide of the following structure: 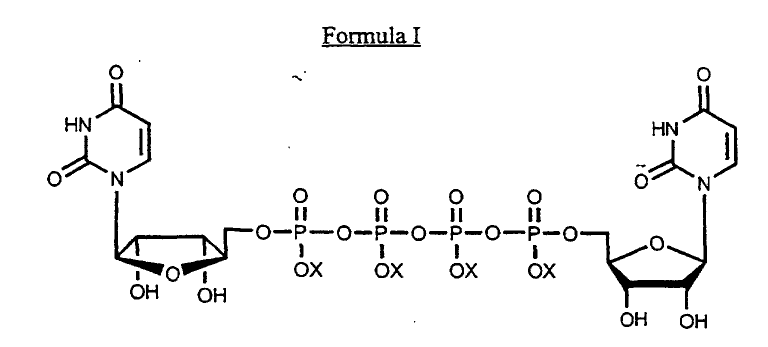 wherein:
X is Na, NH₄ or H, provided that all X groups are not H.

The free acid of P¹,P⁴-di(uridine 5')-tetraphosphate, where X is hydrogen, has been previously described as uridine 5'-(pentahydrogen tetraphosphate), P'''→5'-ester with uridine (CAS Registry Number: 59985-21-6; C. Vallejo et al., *Biochimica et Biophysica Acta* 438, 305 (1976) and H. Coste et al., J. Biol. Chem. 262, 12096 (1987)).

Different methods have been described for the synthesis of purine dinucleotides such as diadenosine tetraphosphate (A₂P₄) (E. Rappaport et al, *Proc. Natl*. *Acad*. *Sci,* 78, 838, (1981); A. Guranowski et al, *Biochemistry*, 27, 2959, (1988); C. Lobaton et al, Eur. J. Biochem., 50, 495, 1975; K. Ng and L. Orgel, *Nucl*. *Acid Res.,* 15, 3573, (1987)). However, this has not been true for U₂P₄ which is a pyrimidine nucleotide. Although purine nucleotides and pyrimidine nucleotides appear to be analogous, the methods used for purine nucleotide synthesis do not necessarily work for pyrimidines such as uridine.

Diuridine tetraphosphate has been shown to have beneficial properties in the treatment of various diseases, such as chronic obstructive pulmonary disease (COPD). For example, they have been demonstrated to facilitate the clearance of mucous secretions from the lungs of a subject such as a mammal including humans in need of treatment for various reasons, including cystic fibrosis, chronic bronchitis, asthma, bronchiectasis, post-operative mucous retention, pneumonia, primary ciliary dyskinesia (M. J. Stutts, III, et al, U.S. Patent No. 5,635,160; PCT International Publication WO 96/40059) and the prevention and treatment of pneumonia in immobilized patients (K. M. Jacobus and H. J. Leighton, U.S. Patent No. 5,763,447). Further therapeutic uses include treatment of cystic fibrosis, chronic bronchitis, asthma, bronchiectasis, post-operative aletectasis and Kartagener's syndrome (PCT International Publication WO 96/40059), sinusitis (PCT International Publication WO 98/03177), otitis media (PCT International Publication WO 97/29756), dry eye, retinal detachment, nasolacrimal duct obstruction, the treatment of female infertility and irritation due to vaginal dryness via increased mucus secretions and hydration of the epithelial surface, and enhancing the performance of athletes.

U₂P₄ also has utility as a veterinary product in mammals such as, but not limited to, dogs, cats and horses.

Prior art methodology describes only one protocol for the production of diuridine tetraphosphate. This method is very time consuming, lasting over five days and producing only small amounts of diuridine tetraphosphate (C. Vallejo et al., *Biochimica et Biophysica Acta* 438, 305 (1976), Sillero et al., *Eur J Biochem* 76, 332 (1972)). According to this technique, diuridine tetraphosphate was synthesized through a reaction of uridine 5'-monophosphomorpholidate (0.54 mmol) with the triethylamine salt of pyrophosphoric acid (0.35 mmol) in a medium of anhydrous pyridine (10 ml). After 5 days at 30° C, pyridine was removed from the reaction mixture by evaporation, and the residue resuspended in glass-distilled water (8 mL), the suspension applied to a DEAE-cellulose column (37.5 x 2.6 cm) and fractionated with 3.2 L of a linear gradient (0.06-0.25 M) of ammonium bicarbonate, pH 8.6. The peak eluting between 0.17-0.19 M ammonium bicarbonate was partially characterized as U₂P₄ by the following criteria: insensitivity to alkaline phosphatase, phosphorus to base ratio and analysis of the products of hydrolysis (UTP + UMP), after treatment with phosphodiesterase I, by electrophoresis in citrate buffer, pH 5.0. No yield or spectroscopic data were given. Thus, the prior art procedure for the synthesis of diuridine tetraphosphate is lengthy and produced only small amounts of only partially characterized diuridine tetraphosphate. The present invention focuses on methods to produce this medically useful compound which may be more efficiently and conveniently carried out, and which may be applied to the large-scale production of diuridine tetraphosphate and salts thereof.

### SUMMARY OF THE INVENTION

The present invention provides new methods for the synthesis of the therapeutic dinucleotide, P¹,P⁴-di(uridine 5')-tetraphosphate (Formula I), and demonstrates applicability to the production of large quantities. The methods of the present invention substantially reduce the time required to synthesize diuridine tetraphosphate, preferably to three days or less. The novel ammonium and sodium salts of P¹,P⁴-di(uridine 5')-tetraphosphate prepared by these methods are stable, soluble, nontoxic, and easy to handle during manufacture. The tetraammonium salt is preferred; the tetrasodium salt is most preferred. 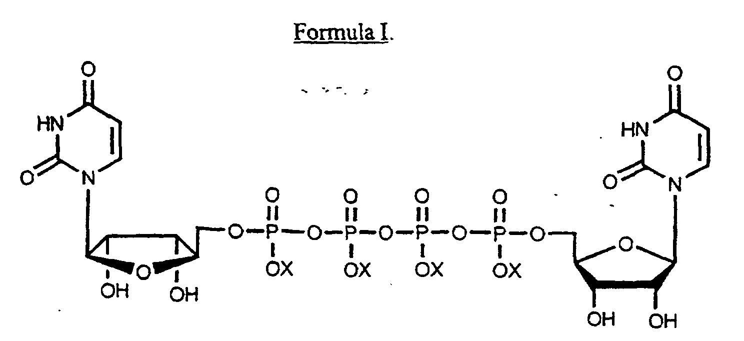 wherein:
X is Na, NH₄ or H, provided that all X groups are not H.

The method of synthesizing compounds of Formula I, and pharmaceutically acceptable salts thereof, is carried out generally by the following steps: 1) dissolving uridine or uridine nucleotide compounds of Formulas IIa-d in a polar, aprotic organic solvent and a hydrophobic amine; 2) phosphorylating with a phosphorylating agent of Formulas IVa-b and/or activating with an activating agent of Formulas IIIa-c; and 3) purifying by ion exchange chromatography.

Another aspect of the present invention are the uses of the compounds of formula I for the manufacture of a medicament for the treatment of various disease states, including, but not limited to: chronic obstructive pulmonary diseases, sinusitis, otitis media, nasolacrimal duct obstruction, dry eye disease, retinal detachment, pneumonia, and female infertility or irritation caused by vaginal dryness.

Another aspect of the present invention is a pharmaceutical composition comprising a compound of Formula I together with a pharmaceutically acceptable carrier.

### DETAILED DESCRIPTION OF THE INVENTION

The present invention provides new methods for the synthesis of the therapeutic dinucleotide, P¹,P⁴-di(uridine 5')-tetraphosphate, and demonstrates applicability to the production of large quantities. The methods of the present invention substantially reduce the time period required to synthesize P¹,P⁴-di(uridine 5')-tetraphosphate, preferably to three days or less. The ammonium and sodium salts of P¹,P⁴-di(uridine 5')-tetraphosphate (Formula I) prepared by these methods are stable, soluble, nontoxic, and easy to handle during manufacture.

The present invention further provides compounds of Formula I: 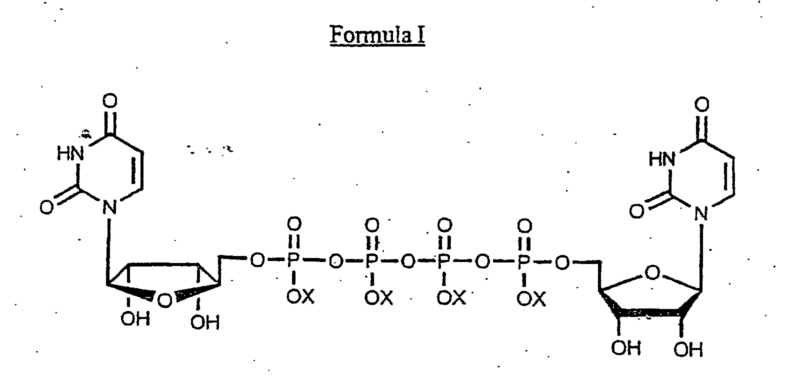 wherein:
X is Na, NH₄ or H, provided that all X groups are not H.

The sodium and ammonium salts of P¹,P⁴-di(uridine 5')-tetraphosphate have many advantages. The sodium and ammonium salts provide good long-term stability profiles compared to those of divalent cations (e.g. Ca²⁺, Mg²⁺, Mn²⁺) which catalyze hydrolysis of phosphate esters. The tetrasodium salt of P¹,P⁴-di(uridine 5')-tetraphosphate is non-irritating to the lung and eyes. Other cations may be irritating to the lungs, eyes, and other mucosal epithelia, or are otherwise not well tolerated by the human body. These inorganic sodium and ammonium salts impart excellent water solubility compared to hydrophobic amine salts such as tri- and tetrabutylammonium, and similar salts. High water solubility is an important advantage for flexibility in pharmaceutical formulations of varying concentration. The tetraammonium and tetrasodium salts of P¹,P⁴-di(uridine 5')-tetraphosphate are also advantageous in that they are readily purified by aqueous ion chromatography in which no organic solvents are used. In addition, these salts are easily handled as fluffy, white solids, compared to an oil or gum as with some amine salts.

The tetrasodium salt is preferred.

The compounds of Formula I may be used to facilitate the clearance of mucous secretions from the lungs of a subject such as a mammal including humans in need of treatment for various reasons, including cystic fibrosis, chronic bronchitis, asthma, bronchiectasis, post-operative mucous retention, pneumonia, primary ciliary dyskinesia (M. J. Stutts, III, et al, U.S. Patent No. 5,635,160; PCT International Publication WO 96/40059) and the prevention and treatment of pneumonia in immobilized patients (K. M. Jacobus and H. J. Leighton, U.S. Patent No. 5,763,447). Further therapeutic uses include treatment of sinusitis (PCT International Publication WO 98/03177), otitis media (PCT International Publication WO 97/29756), dry eye, retinal detachment, nasolacrimal duct obstruction, the treatment of female infertility and irritation due to vaginal dryness via increased mucus secretions and hydration of the epithelial surface, and enhancing the performance of athletes.

The compounds of Formula I may be administered orally, topically, parenterally, by inhalation or spray, intra-operatively, rectally, or vaginally in dosage unit formulations containing conventional non-toxic pharmaceutically acceptable carriers, adjuvants and vehicles. The term topically as used herein includes patches, gels, creams, ointments, suppositiories, pessaries, or nose, ear or eye drops. The term parenteral as used herein includes subcutaneous injections, intravenous, intramuscular, intrasternal injection or infusion techniques. In addition, there is provided a pharmaceutical formulation comprising a compound of general Formula I and a pharmaceutically acceptable carrier. One or more compounds of general Formula I may be present in association with one or more non-toxic pharmaceutically acceptable carriers or diluents or adjuvants and, if desired, other active ingredients. One such carrier would be sugars, where the compounds may be intimately incorporated in the matrix through glassification or simply admixed with the carrier (e.g., lactose, sucrose, trehalose, mannitol) or other acceptable excipients for lung or airway delivery.

One or more compounds of general Formula I may be administered separately or together, or separately or together with: mucolytics such as DNAse (Pulmozyme®) or acetylcysteine, antibiotics, including but not limited to inhaled Tobramycin®; non-steroidal anti-inflammatories, antivirals, vaccines, decongestants and corticosteroids.

The pharmaceutical compositions containing compounds of general Formula I may be in a form suitable for oral use, for example, as tablets, caplets, lozenges, aqueous or oily suspensions, dispersible powders or granules, emulsion, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water or an oil medium, for example, peanut oil, liquid paraffin or olive oil.

Aqueous suspensions contain the active materials in admixture with excipients suitable for the manufacture of aqueous suspensions. Such excipients are suspending agents, for example: sodium carboxymethylcellulose, methylcellulose and sodium alginate. Dispersing or wetting agents may be a naturally-occurring phosphatide or condensation products of an allylene oxide with fatty acids, or condensation products of ethylene oxide with long chain aliphatic alcohols, or condensation products of ethylene oxide with partial esters from fatty acids and a hexitol, or condensation products of ethylene oxide with partial esters derived from fatty acids and hexitol anhydrides. Those skilled in the art will recognize the many specific excipients and wetting agents encompassed by the general description above. The aqueous suspensions may also contain one or more preservatives, for example, ethyl, or n-propyl p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredients in admixture with a dispersing or wetting agent, suspending agent and one or more preservatives. Suitable dispersing or wetting agents and suspending agents are exemplified by those already mentioned above. Additional excipients, for example, sweetening, flavoring, and coloring agents, may also be present.

Compounds of Formula I may be administered parenterally in a sterile medium. The drug, depending on the vehicle and concentration used, can either be suspended or dissolved in the vehicle. Advantageously, adjuvants such as local anaesthetics, preservatives and buffering agents can be dissolved in the vehicle. The sterile injectable preparation may be a sterile injectable solution or suspension in a non-toxic parentally acceptable diluent or solvent. Among the acceptable vehicles and solvents that may be employed are sterile water, saline solution, or Ringer's solution. The compounds of general Formula I may also be administered in the form of suppositories for ear, rectal or vaginal administration of the drug. These compositions can be prepared by mixing the drug with a suitable non-irritating excipient which is solid at ordinary temperatures but liquid at the body temperature and will therefore melt to release the drug. Such materials are cocoa butter and polyethylene glycols.

Solutions of compounds of Formula I may be administered by intra-operative installation at any site in the body.

Single dosage levels of the order of from about 1 to about 400 mg, preferably in the range of 10 to 300 mg, and most preferably in the range of 25 to 250 mg, are useful in the treatment of the above-indicated respiratory conditions. Single dosage levels of the order of from about 0.0005 to about 5 mg, preferably in the range of 0.001 to 3 mg and most preferably in the range of 0.025 to 1 mg, are useful in the treatment of the above-indicated ophthalmic conditions. The amount of active ingredient that may be combined with the carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

The synthetic methods described below encompass several synthetic strategies for producing P¹,P⁴-di(uridine 5')-tetraphosphate. Generally, all the methods use uridine or uridine nucleotide compounds from Formula IIa-d as starting materials, which are dissolved in a polar, aprotic organic solvent (e.g. dimethylformamide, dimethylsulfoxide, dioxane, N-methylpyrrolidone, trimethylphosphate) and a hydrophobic amine (e.g. triethylamine, tributylamine, trioctylamine, 2,4,6-collidine, tetrabutylammonium, tri- and tetra-alkyl amines, heterocyclic amines). The product is obtained by phosphorylating with a phosphorylating agent from Formula IV (e.g. phosphorus oxychloride, pyrophosphate, pyrophosphorylchloride) or activating a phosphate group with an activating agent from Formula III (e.g. carbonyldiimidazole, an alky or aryl carbodiimide, an alkyl or aryl phosphochloridate), respectively, with subsequent purification various means well known to those of skill in the art, including, but not limited to, ion chromatography (e.g. DEAE Sephadex, DEAE cellulose, Dowex 50, anion and cation exchange resins).

The pyrimidine β-D-ribofuranosyl starting materials uridine, uridine 5'-monophosphate (UMP), uridine 5'-diphosphate (UDP), and uridine 5'-triphosphate (UTP) are shown as free acids in Formulas IIa-d below, respectively. These materials are all commercially available in large quantity in various salt forms. 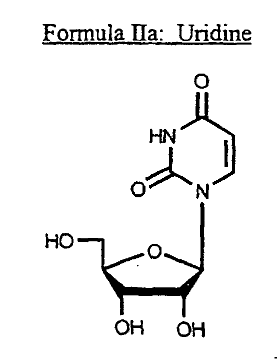 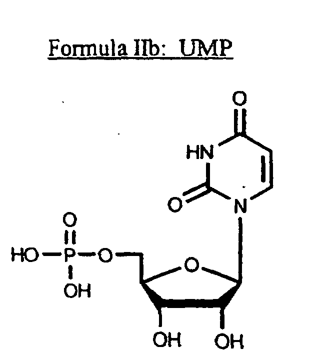 and salts thereof; 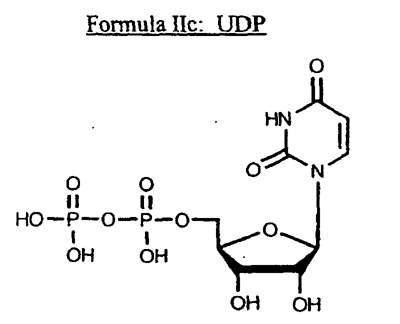 and salts thereof; 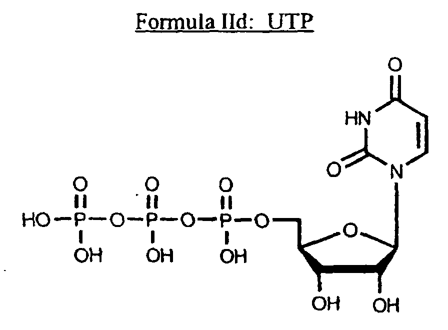 and salts thereof.

The activating agents carbodiimide, activated carbonyl, and activated phosphorus compounds are shown in the general Formulas IIIa-c below, respectively. 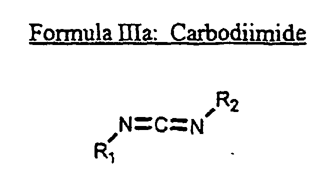 wherein R₁ and R₂ are C₁-C₈ alkyl or cycloalkyl, C₁-C₈ optionally substituted alkyl or cycloalkyl (e.g..hydroxy and amino groups); aryl or optionally substituted aryl (e.g. hydroxy and amino groups). Preferred compounds of Formula IIIa are dicyclohexylcarbodiimide and 1-(3-Dimethylaminopropyl)-3-ethylcarbodiimide hydrochloride. 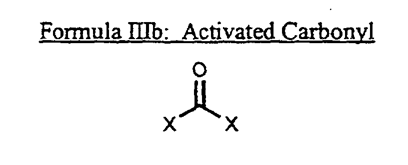 wherein X is imidazole, tetrazole, and/or halogen. Preferred compounds of Formula IIIb are carbonyldiimidazole and carbonylditriazole. 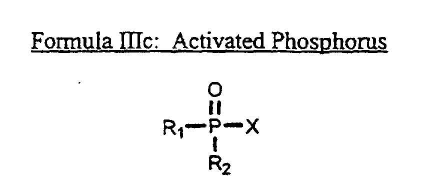 wherein R₁ and R₂ are C₁-C₈ alkyl or cycloalkyl, C₁-C₈ optionally substituted alkyl, alkoxy or cycloalkyl (e.g. hydroxy and amino groups); aryl, aryloxy, alkoxy or optionally substituted aryl, aryloxy, or alkoxy (e.g. hydroxy and amino groups) and/or halogen; and X is halogen. Preferred compounds of Formula IIIc are diphenylphosphorochloridate, phenyl phosphorodichloridate, phenylphosphonic dichloride and diphenylphosphinic chloride.

The mono- and diphosphorylating agents are shown below in the general formulas IVa-b. 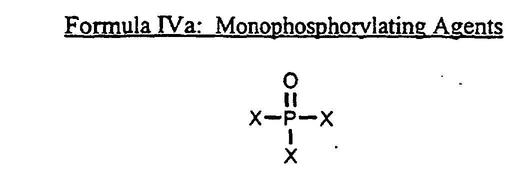 wherein X is halogen. Preferred compound of Formula IVa is phosphorus oxychloride. 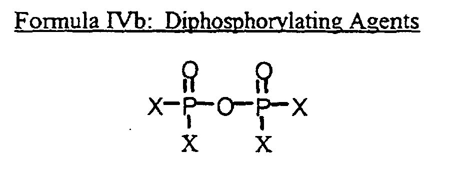 wherein X is oxygen, hydroxy, or halogen, and salts thereof. Preferred compounds of Formula IVb are pyrophosphoryl chloride and pyrophosphate.

Those having skill in the art will recognize that the present invention is not limited to the following examples and that the steps in the following examples may be varied.

### EXAMPLE 1

### Method for the Production of Diuridine Tetraphosphate, Tetrasodium Salt Using Uridine 5'-Diphosphate

Uridine 5'-diphosphate disodium salt (Yamasa, Choshi, Japan; 600 grams) was dissolved in deionized water (5.4 L). The solution was passed through a Dowex 50Wx4 H⁺ (Dow Chemical) column. The fractions containing undine 5'-diphosphate were pooled and neutralized with tributylamine (Aldrich, St. Louis; 300 mL). The neutralized fractions were concentrated to an oil by using a rotary evaporator at a bath temperature of 55-60° C. The oil was dissolved in dry dimethylformamide (Aldrich, 3 L) and then dried by concentrating to an oil using a rotary evaporator (55-60° C bath temperature). This step was repeated twice. The oil was again dissolved in dimethylformamide (3 L) and 1,1-carbonyldiimidazole (Aldrich; 100g) was added. The solution was heated at 50° C for 2½ hours. An additional amount of activating agent (33 grams) was added and heating continued for a further 2½ hours. The solution was again concentrated to an oil on a rotary evaporator (bath temperature at 55-60° C). The resulting oil was dissolved in deionized water to a conductivity equal to that of 0.2 M NH₄HCO₃. The solution was then loaded into a column of Sephadex DEAE-A25 (Pharmacia, Upsala, Sweden; pre-swollen in 1.0 M NaHCO₃ and washed with 2 column volumes of deionized H₂O). The column was eluted with the following solutions in the following order: 60 L of 0.25 M NH₄HCO₃, 120 L of 0.275M NH₄HCO₃, 40 L of 030 M NH₄HCO₃ and 40 L of 0.35 M NH₄HCO₃. The fractions having sufficient amounts of pure diuridine tetraphosphate were pooled as determined by HPLC analysis and concentrated on a rotary evaporator (bath temperature at 55-60° C). The resulting residue was dissolved in deionized water (1.5 L) and concentrated on a rotary evaporator. This step was repeated 15 times or until excess of bicarbonate buffer was removed. The resulting oil was dissolved in a sufficient amount of deionized water to form a ca. 10% solution, the solution charged to a Dowex 50Wx4 Na⁺ (Dow) column and eluted with deionized water. The fractions containing U₂P₄ were pooled and concentrated to a ca. 10-15% solution, which was lyophilized to yield U₂P₄ tetrasodium salt as a white solid (150g approximately 25% yield based on uridine 5'-diphosphate).

### Structure Elucidation of P¹,P⁴-di(uridine 5')-tetraphosphate, Tetrasodium Salt

Due to the lack of adequate spectroscopic data of nonadenylated dinucleotides in the literature, a full structure elucidation of P¹,P⁴-di(uridine 5')-tetraphosphate, tetrasodium salt was performed by employing modern analytical techniques. The molecular weight was determined by mass spectrometry to be 878 [m/z 855, (M-Na⁺)⁻], confirming the molecular formula C₁₈H₂₂N₄O₂₃P₄•4Na. The exact mass measured for C₁₈H₂₂N₄O₂₃P₄•3Na [(M-Na⁺)⁻: calculated 854.9318] was 854.9268. The measured mass differed from the theoretical mass by 5.0 milimass units (5.9 ppm) for a confidence level of 99.7%. Karl Fisher moisture analysis gave a value of 1.73% H₂O and further confirmation of the molecular formula was obtained from elemental analysis: calculated for Na = 10.70, found 10.81%; C:P ratio calculated 1.74, found 1.80, based on the molecular formula: C₁₈H₂₂N₄O₂₃P₄•4.2Na•1.1H₂O (FW = 902.4 g/mol). The infrared spectrum showed a broad signal at 3422 cm⁻¹ and a signal at 1702 cm⁻¹, indicating the presence of hydroxyl (O-H stretch) and carbonyl (C=O stretch) functional groups. In addition, a phosphate P=O stretch was observed at 1265 cm⁻¹. The UV spectrum in water displayed a λₘₐₓ of 262 nm with a molar absorptivity (ε) of 17,004. The specific rotation at 25° C (c = 1, H₂O) was determined by polarimetry to be -9.56°.

The NMR spectra are: ¹H NMR (D₂O, TMS) δ 4.11 (m, 2H), 4.14 (m, 1H), 4.25 (m, 1H), 4.27 (m, 1H), 5.84 (d, J=8.1 Hz, 1H), 5.86 (d, J=5.4 Hz, 1H), 7.81 (d, J=8.1 Hz); ¹³C NMR (D₂O, TMS) δ 65.1 (d, J=5.5 Hz), 69.7, 73.5, 83.4 (d, J=9.4 Hz), 88.1, 102.8, 141.5, 152.9, 167.5; ³¹P NMR (D₂O, H₃PO₄ std) δ -22,32 (m),-10.75 (m). The ¹H coupled ³¹P spectrum showed a broadening of the multiplet at δ -10.75 ppm due to the introduction of ¹H coupling. This multiplet was therefore confirmed as P_{α}. There was no effect of ¹H coupling on the multiplet at -22.23 ppm, assigning this by default as P_{β}. A Nuclear Overhauser Effect (NOE) was observed for H₆ to the H₂. and H₃, sugar protons. Because it is not possible for H₅ to show an NOE to the sugar protons, H₆ is confirmed. Additionally, N₁ substitution is confirmed, because no pyrimidine-sugar NOE is possible for an N₃ substituted structure.

Additional 2-dimensional NMR experiments were conducted to verify connectivity. HMQC shows connectivity for H₅ to C₅ and H₆ to C₆, confirming C₅ and C₆. COSY and NOE connectivity were observed for H₅ to H₆, verifying H₅. HMBC 3-bond connectivity was observed for: H₆ to C_{1'}, C₆ to H_{1'}, H_{1'} to C₂, H₆ to C₂. These data thus confirm H₁, C₂ and N₁ substitution. COSY connectivity of H_{1'} to H_{2'} confams H_{2'} and HMQC connectivity of H_{1'} to C_{1'} and H_{2'} to C_{2'} confirms C_{1'} and C_{2'}. Additionally, HMBC shows 2-bond J connectivity from H₅ to C₄, confirming C₄. A ¹³C DEPT spectrum with mult = 1.5 shows the carbon at δ 65.1 inverted relative to all other carbons. This observation confirms that C_{5'} is a methylene. The coupling of ³¹P to carbons at δ 65.1 and 83.4 confirms C_{5'} and C_{4'}, because C_{4'} is the only coupled methyne. In addition, HMQC shows connectivity for C_{5'} to H_{5'} and C_{4'} to H_{4'}, confirming H_{4'} and H_{5'}. An NOE was observed for H_{1'} to H_{4'}, H₆ to H_{2'} and H₆ to H_{3'}, confirming the β anomer sugar configuration.

In conclusion, P¹,P⁴-di(uridine 5')-tetraphosphate, tetrasodium salt was synthesized on a 150 g scale in 25% yield from commercially available starting materials with a total reaction time of 5 hours. The crude product was efficiently purified by ion exchange chromatography and the structure of the reaction product was unambiguously proven using mass spectroscopic, NMR and other analytical techniques.

### EXAMPLE 2

### Method for the Production of Diuridine Tetraphosphate Tetraammonium Salt Using Uridine 5'-Monophosphate

Uridine 5'-monophosphate (Sigma, Milwaukee, 3.0g, 9.26mmol) was dissolved in dry DMF (10 mL) and tributylamine (Aldrich, 2 mL). The solution was evaporated *in vacuo* at 40° C to an oil. The residue was dissolved in dry DMF (Aldrich, 8 mL) to form a solution. Carbonyldiimidazole (Aldrich, 1.65 g, 10.18 mmol) was added to this solution. The reaction was heated at 50° C for one hour. Uridine 5'-triphosphate (Yamasa, 5.60 g, 10.18 mmol) prepared as the anhydrous tributylammonium salt in DMF (5 mL) and tributylamine (2 mL), as described in Example 3 below, was added to the reaction solution. The mixture was allowed to stir at 50°C for three days when the solution was evaporated *in vacuo* to an oil, redissolved in water (5 mL) and purified by column (300 X 50 mm) chromatography (Sephadex DEAE-A25, 40-120µ, Aldrich, pre-swollen in 1.0 M NaHCO₃ and washed with 2 column volumes of deionized H₂O (H₂O→0.3 M NH₄HCO₃ gradient). The pure fractions were concentrated *in vacuo* at 35° C, and H₂O added and reevaporated 5 times to obtain diuridine tetraphosphate tetraammonium salt as a white solid (2.37 g, 30% yield): 92.11% pure by HPLC with the same retention time as the standard. In addition, the tetraammonium salt was analyzed by FABMS to give a mass of [C₁₈H₂₅N₄O₂₃P₄ (M-H⁺)⁻: calculated 788.9860] 788.9857, confirming a parent formula of C₁₈H₂₆N₄O₂₃P₄ for the free acid].

### EXAMPLE 3A

### Method for the Production of Diuridine Tetraphosphate Tetraammonium Salt Using Uridine 5'-Triphosphate (UTP)

A solution of uridine 5'-triphosphate (UTP) trisodium salt (ProBioSint, Varese, Italy; 5.86 g, 0.01 mol) in water (5 mL) was passed through a column of BioRad AG-MP 50 (Aldrich) strong cation exchange resin in its tributylamine form (50 mL bed volume) and eluted with distilled water (about 300 mL). To this solution was added tributylamine (Aldrich; 5 mL), and the suspension shaken until the pH of the aqueous fraction had risen to 8. The layers were separated and the aqueous solution evaporated to small volume, then lyophilized overnight. The residue was dissolved in dry dimethylformamide (Aldrich; 20 mL) and the solvent evaporated at 0.1 mmHg. The dried tributylamine salt was made up to 100 mL with anhydrous acetone to yield a stock solution (0.1 M in UTP). Dicyclohexylcarbodiimide (DCC) (Baker, Phillipsburg; 0.227 g, 1.2 mmol) was added to an aliquot of the foregoing UTP solution (10 mL, 1.0 mmol) and the solution stirred at room temperature for 30 minutes. The mixture was added to the triethylamine salt of uridine 5'-monophosphate (2.0 mmol, prepared by addition of triethylamine (0.5 mL) to a solution of uridine 5'-monophosphate (UMP) (Sigma; 0.648 g in DMF), and evaporating to dryness). This suspension was then evaporated to dryness, the residue made up to 5.0 mL in dry DMF, and set aside at 40° C for 24 hours. The reaction mixture was separated by semipreparative ion-exchange chromatography (Hamilton PRP X-100 column), eluting with a gradient of 0-1.0 M ammonium bicarbonate, 5 mL/min, 30 minutes. The dinucleotide tetraphosphate eluted between 21 and 23 minutes; the product (76.7% yield based on UTP) was quantitated by comparison of its ultraviolet absorption at λₘₐₓ 263 nm with that of a standard solution of P¹,P⁴-di(uridine 5')-tetraphosphate.

### EXAMPLE 3B

### Method for the Production of Diuridine Tetraphosphate Tetraammonium Salt Using Uridine 5'-Triphosphate (UTP) and an Excess of Activating Agent

Conversion of UTP to P¹,P⁴-di(uridine 5')-tetraphosphate can be enhanced by activation of the tributylamine salt (0.1 mmol) with a large excess of DCC (0.1g, .5 mmol); in this case the deposited dicyclohexylurea was removed by filtration, the reaction mixture extracted with ether (10 mL) and the residue dissolved in dry DMF prior to treatment with tributylamine UMP (0.2 mmol). Upon chromatographic separation of the reaction mixture and quantitation by ultraviolet absorption as in Example 3A above, the uridine tetraphosphate product constituted 50.7% of the uridylate species in the mixture, corresponding to a conversion from UTP of 95.9%.

### EXAMPLE 4A

### Method for the Production of Diuridine Tetraphosphate Tetraammonium Salt Using Uridine 5'-Monophosphate Activated with Carbonyldiimidazole

Uridine 5'-monophosphate (UMP) (0.324g, 1.0 mmol) was dissolved in a mixture of dry DMF (5 mL) and tributylamine (237 µL, 1 mmol) the solution was evaporated to dryness, then twice more with DMF to yield the anhydrous tributylamine salt. The residue was dissolved in DMF (5 mL) and carbonyldiimidazole (CDI) (0.81 g, 5 mmol) added. The solution was set aside for 3 hours, then methanol 324 µL, 8 mmol) added to destroy the excess of CDI. The solution was set aside for one hour. Tributylamine pyrophosphate (Sigma, 0.228 g, 0.5 mmol) was added and the suspension stirred under nitrogen at room temperature. After 3 hours the reaction was quenched with water and the mixture subjected to HPLC as in Example 3A above. Yield of P¹,P⁴-di(uridine 5')-tetraphosphate as quantitated by its absorbance at 263 nm was 9.3%.

### EXAMPLE 4B

### Method for the Production of Diuridine Tetraphosphate Tetraammonium Salt Using Uridine 5'-Monophosphate Activated with Diphenyl Phosphochloridate

The anhydrous tributylamine salt of UMP (1.0 mmol), prepared essentially as above, was dissolved in a mixture of dry dioxane (5 mL) and DMF (1 mL). Diphenyl phosphochloridate (0.3 mL) and tributylamine (0.3 mL) were added and the solution set aside at room temperature for 3 hours. The solvent was evaporated and the residue shaken with ether (∼10 mL), then set aside at 4° C for 30 minutes. The ether was decanted and the residue was dissolved in a solution of tributylamine pyrophosphate (0.228 g, 0.5 mmol) in DMF (3 mL). The solution was stored under nitrogen at room temperature. After 3 hours the reaction was quenched with water and the mixture subjected to HPLC as in Example 3A above. Yield of P¹,P⁴-di(uridine 5')-tetraphosphate as quantified by its absorbance at 263 nm was 9.6%.

### EXAMPLE 5

### Method for the Production of Diuridine Tetraphosphate Tetraammonium Salt Using Uridine, Phosphorus Oxychloride and Pyrophosphate

Uridine (Aldrich, 0.244 g, 1 mmol) was dissolved in trimethyl phosphate (Aldrich, 5 mL) and tributylamine (466 uL, 2 mmol) added. The solution was stirred at 0 degrees during the addition of phosphorus oxychloride (0.153 g (93.2 uL), 1 mmol), and the resulting suspension stirred at 0° C for 3 hours. Tributylamine pyrophosphate (0.228 g) was added and the suspension stirred at room temperature for 3 hours. The reaction was quenched with 1.0 M aqueous triethylamine bicarbonate and the mixture extracted with methylene chloride to remove trimethyl phosphate. The aqueous solution was subjected to HPLC as in Example 3A above. Conversion of uridine to P¹,P⁴-di(uridine 5')-tetraphosphate as quantitated by absorbance of the latter at 263 nm was 6.83%.

### EXAMPLE 6

### Method for the Production of Diuridine Tetraphosphate Using Uridine 5'-Monophosphate and Pyrophosphoryl Chloride

Uridine 5'-monophosphate (UMP) (64.8 mg, 0.2 mmol) was dissolved in dry pyridine (1 mL) and stirred in ice during the addition of pyrophosphoryl chloride (13.9 uL (25 mg), 0.1 mmol). The solution became cloudy almost immediately, then a copious semicrystalline white precipitate formed which became a gummy mass within 1-2 minutes. The mixture was stored at room temperature overnight, the quenched with water and subjected to HPLC as in Example 3A above. Yield of P¹,P⁴-di(uridine 5')-tetraphosphate as quantitated by its absorbance at 263 nm was 15.8%. A substantial amount of P¹,P³-di(uridine 5')-triphosphate (25.4%) was obtained as the major by-product.

### EXAMPLE 7

### Aqueous Stability and Solubility of P¹,P⁴-di(uridine 5')-tetraphosphate, Tetrasodium salt

The solubility of P¹,P⁴-di(uridine 5')-tetraphosphate, tetrasodium salt in water was determined by adding portions of solid to a known volume of deionized water until the solution became turbid. The maximum solubility in water was thus determined to be ca. 900 mg/mL. Stability studies of the solid or aqueous solutions incubated at low (5° C) and elevated temperatures (40° C) showed that less than 1.5% degradation occurs over a three month period as determined by HPLC analysis. The tetrasodium salt of P¹,P⁴-di(uridine 5')-tetraphosphate was thus determined to have an excellent solubility and stability profile suitable for pharmaceutical applications.

### EXAMPLE 8

### Toxicity of P¹,P⁴-di(uridine 5')-tetraphosphate, Tetrasodium Salt in Animals

The nonclinical toxicologic profile of P¹,P⁴-di(uridine 5')-tetraphosphate, tetrasodium salt has been evaluated in a battery of genetic toxicology assays that include the bacterial reverse mutation assay, the *in vitro* mammalian cytogenetic test, the *in vitro* mammalian cell gene mutation test, and the micronucleus cytogenetic assay in mice. A study in rabbits examined local ocular tolerance and subchronic ocular toxicity after multiple daily administrations over a six-week period. In addition, P¹,P⁴-di(uridine 5')-tetraphosphate, tetrasodium salt has also been tested in two single-dose acute inhalation toxicity studies in rat and dog, and one single-dose acute intravenous toxicity study in dogs.

The results of these studies show that P¹,P⁴-di(uridine 5')-tetraphosphate, tetrasodium salt is nongenotoxic in a battery of genetic toxicology assays. No adverse findings were seen in the ocular toxicology studies. A low degree of acute toxicity was seen in single dose inhalation (rats, dogs) and intravenous (dogs) toxicity studies. P¹,P⁴-di(uridine 5')-tetraphosphate, tetrasodium salt was therefore determined to have an excellent toxicology profile with a wide safety margin for dosing in humans.

### EXAMPLE 9

### Safety and Efficacy of P¹,P⁴-di(uridine 5')-tetraphosphate, Tetrasodium Salt in Normal Human Volunteers

P¹,P⁴-di(uridine 5')-tetraphosphate, tetrasodium salt was evaluated in a Phase I, double-blind, placebo-controlled, escalating dose, safety and tolerability study in 75 normal healthy male volunteers. Forty non-smokers and 35 smokers were evaluated in 5 dosing cohorts of 16 volunteers, comprised of 12 receiving a single aerosolized dose of P¹,P⁴-di(uridine 5')-tetraphosphate, tetrasodium salt (20-400 mg) and 4 receiving placebo (normal saline). No serious adverse events were reported. There were no significant changes in FEV₁, FVC, MMEF, clinical laboratory, 12-lead ECG, or urinalysis results in either the placebo or active drug groups. In smokers, P¹,P⁴-di(uridine 5')-tetraphosphate, tetrasodium salt produced a 2-fold to 7-fold dose-dependent increase in the weight of sputum expectorated within 5 minutes of dosing, and stimulation of sputum expectoration was sustained over the next hour of sputum collection. The effect of P¹,P⁴-di(uridine 5')-tetraphosphate, tetrasodium salt to induce the expectoration of sputum in non-smokers was also observed. In conclusion, P¹,P⁴-di(uridine 5')-tetraphosphate, tetrasodium salt is safe and well-tolerated in normal male subjects and is effective in stimulating the expectoration of sputum when compared to placebo.

## Claims

1. P¹,P⁴-di(uridine 5')-tetraphosphate, tetrasodium salt.

2. A composition comprising at least 92% pure P¹,P⁴-di(uridine 5')-tetraphosphate, tetrammonium salt.

3. A process for the synthesis of compounds of Formula I, and pharmaceutically acceptable salts thereof, said process comprising:
a) dissolving uridine or a uridine nucleotide compound of Formulas IIa-d in a polar, aprotic organic solvent and a hydrophobic amine;
b) phosphorylating with a phosphorylating agent of one of the Formulas IVa-b to yield a compound of Formula I or activating a phosphate group of said uridine nucleotide compound with an activating agent of one of the Formulas IIIa-c; and reacting with a suitable compound of Formula II b-d to yield a compound of Formula I; and
c) purifying by ion exchange chromatography said compound of Formula I, and pharmaceutically acceptable salts thereof;
wherein 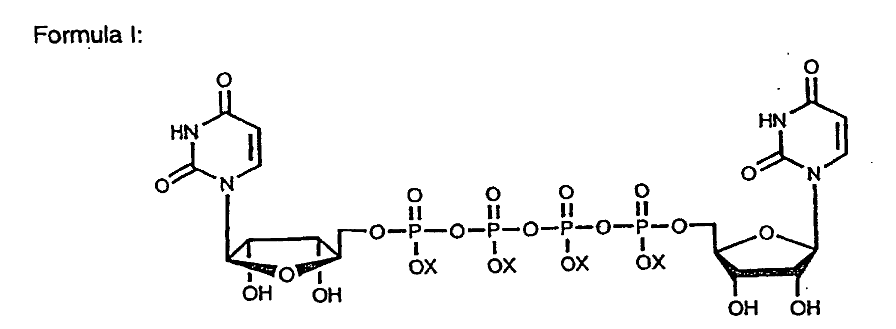 wherein X is selected from the group consisting of: Na, NH₄ and H, provided that all X groups are not H; 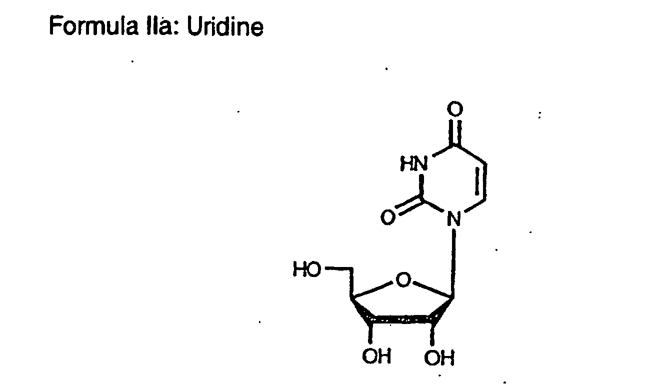 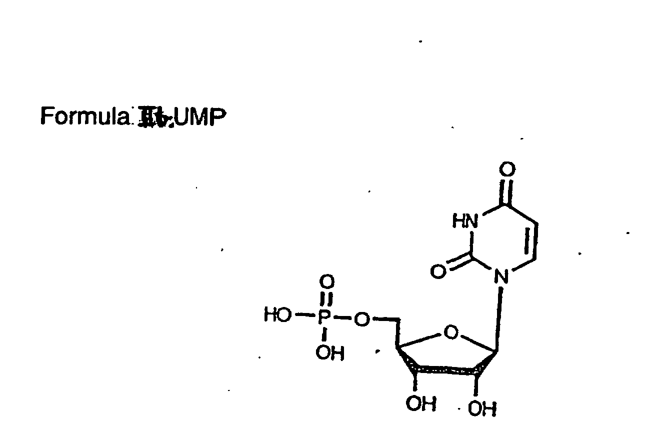 and salts thereof; 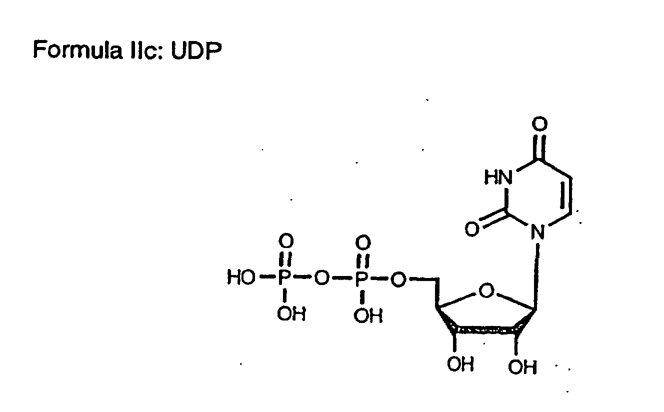 and salts thereof; 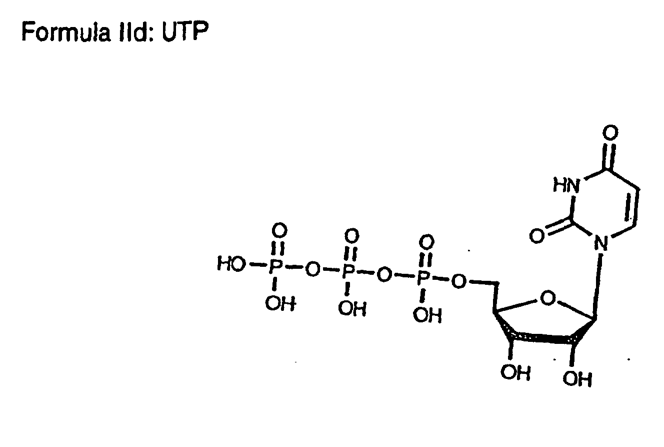 and salts thereof; 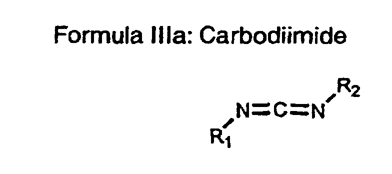 wherein R₁ and R₂ are independently selected from the group consisting of: C₁-C₈ alkyl, cycloalkyl, and aryl wherein said alkyl, cykloalkyl, or aryl is optionally substituted with hydroxyl, or amino groups; 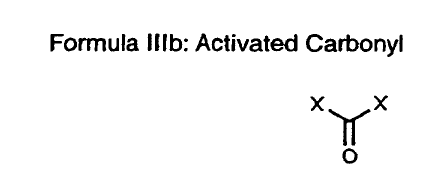 wherein X is independently selected from the group consisting of: imidazole, tetrazole and halogen; 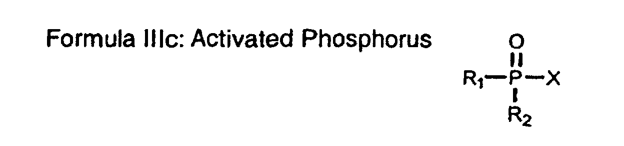 wherein X is halogen and R₁ and R₂ are each independently selected from the group consisting of: C₁-C₈ alkyl, cycloalkyl, C₁-C₈ alkoxy, cycloalkoxy, aryl, aryloxy, and halogen, wherein said alkyl, cycloalkyl, alkoxy, cycloalkoxy, aryl or aryloxy is optionally substituted with hydroxyl, amino, or halogen groups; 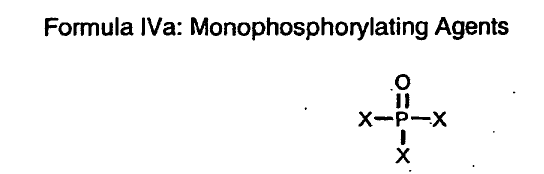 wherein X is halogen; 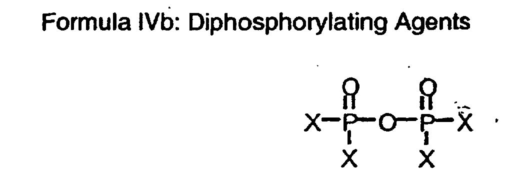 wherein X is selected from the group consisting of: oxygen, hydroxy, halogen, and salts thereof.

4. The process of claim 3, wherein the compounds of Formula IIIa are selected from the group consisting of: dicyclohexylcarbodiimide and 1-(3 -dimethylaminopropyl)-3-ethylcarbodiimide hydrochloride.

5. The process of claim 3, wherein the compounds of Formula IIIb are selected from the group consisting of: carbonyldiimidazole and carbonylditriazole.

6. The process of claim 3, wherein the compound of Formula IIIc is selected from the group consisting of: diphenylphosphorochloridate, phenyl phosphorodidhloridate, phenylphosphonic dichloride and diphenylphosphinic chloride.

7. The process of claim 3, wherein the compound of Formula IVa is phosphorus oxychloride.

8. The process of claim 3, wherein the compound of Formula IVb is selected from the group consisting of: pyrophosphoryl chloride and pyrophosphate.

9. The process according to claim 3, comprising dissolving a compound of Formula IIc as described in claim 3 in a polar, aprotic organic solvent and a hydrophobic amine, treating with an activating agent from one of the Formulas IIIa-c as described in claim 3, and subsequent purification by chromatography.

10. A process for the preparation of compounds of Formula I: 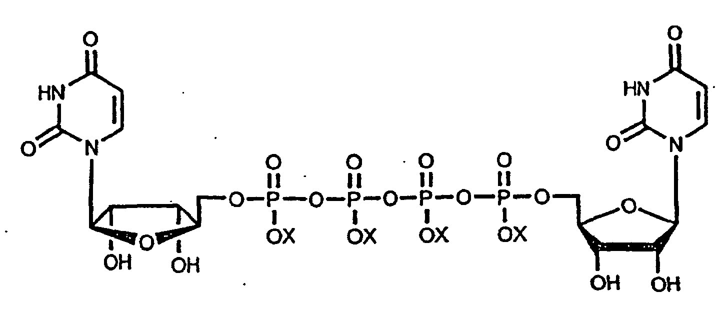 wherein:
X is selected from the group consisting of: Na, NH₄ and H, provided that all X groups are not H;
and pharmaceutically acceptable salts thereof, said process comprising dissolving a compound of Formula IIb as described in claim 3 in a polar, aprotic organic solvent and a hydrophobic amine, treating with an activating agent from Formulas IIIa-c as described in claim 3, followed by reacting with a compound of Formula IId as described in claim 3, and subsequent purification by chromatography.

11. The process according to claim 3, comprising dissolving a compound of Formula IId as described in claim 3 in a polar, aprotic organic solvent and a hydrophobic amine, treating with an equimolar amount of activating agent from one of the Formulas IIIa-c as described in claim 3, followed by reacting with a compound of Formula IIb as described in claim 3, and subsequently purification by chromatography.

12. The process according to Claim 11 whereby an excess of activating agent is used.

13. The process according to claim 3, comprising dissolving a compound of Formula IIb as described in claim 3 in a polar, aprotic organic solvent and a hydrophobic amine, treating said compound with an activating agent from one of the Formulas IIIa-c as described in claim 3, followed by reacting with a suitable compound of Formula IVb as described in claim 3, and subsequent purification by chromatography.

14. A process for the preparation of compounds of Formula I: 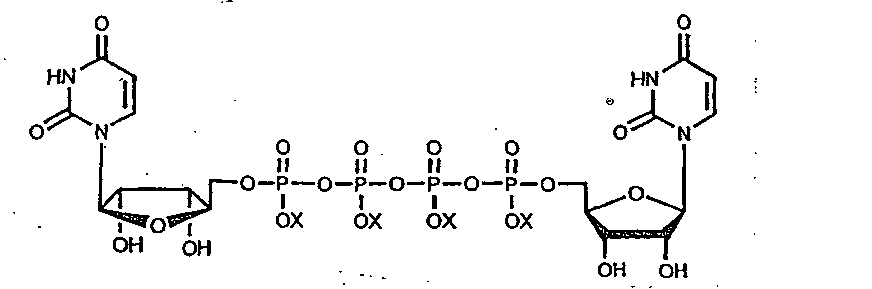 wherein:
X is selected from the group consisting of: Na, NH₄ and H, provided that all X groups are not H;
and pharmaceutically acceptable salts thereof, said process comprising dissolving a compound of Formula IIa as described in claim 3 in a polar, aprotic organic solvent and a hydrophobic amine, treating with phosphorylating agents from Formulas IVa-b as described in claim 3, and subsequent purification by chromatography.

15. The process according to claim 3, comprising dissolving a compound of Formula IIb as described in claim 3 in a polar, aprotic organic solvent and a hydrophobic amine, treating with a phosphorylating agent from Formula IVb as described in claim 3, and subsequent purification by chromatography.

16. Use of a compound for the manufacture of a medicament for the treatment of a condition selected from the group consisting of chronic pulmonary diseases in a mammal, nasolacrimal duct obstruction in a mammal, retinal detachment in a mammal, facilitating sputum induction in a mammal, facilitating expectoration in a mammal and female infertility or vaginal irritation due to vaginal dryness, wherein said compound has the Formula 1: 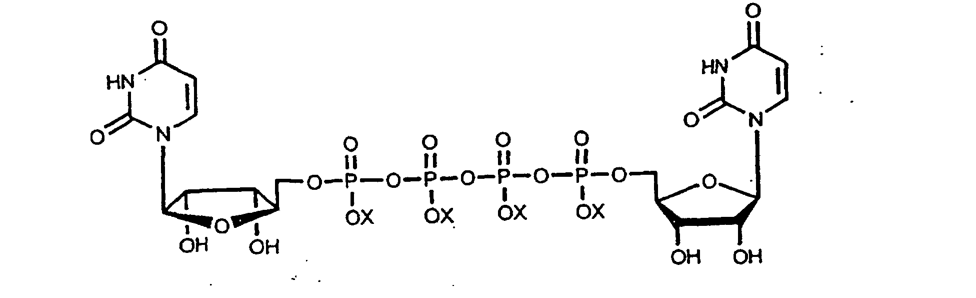 wherein:
X is selected from the group consisting of: Na, NH₄ and H, provided that all X groups are not H.

17. A pharmaceutical composition comprising P¹,P⁴-di(uridine 5')-tetraphosphate, tetrasodium salt together with a pharmaceutically acceptable carrier therefore.

18. The pharmaceutical composition according to Claim 17, wherein said pharmaceutical composition is sterile.

19. The pharmaceutical composition according to Claim 17, wherein the pharmaceutical composition is in the form of a gel, an aqueous suspension, a granule, or powder.

## Patentansprüche

1. P¹,P⁴-Di(uridin-5')-tetraphosphat, Tetranatriumsalz.

2. Zusammensetzung, umfassend mindestens 92 % reines P¹,P⁴-Di(uridin-5')tetraphosphat, Tetrammoniumsalz.

3. Verfahren zur Synthese von Verbindungen der Formel I und pharmazeutisch annehmbaren Salzen davon, wobei das Verfahren folgendes umfasst:
a) Auflösen von Uridin oder einer Uridinnukleotidverbindung der Formeln IIa-d in einem polaren, aprotischen organischen Lösungsmittel und einem hydrophoben Amin;
b) Phosphorylieren einer der Formeln IVa-b mit einem Phosphorylierungsmittel, um eine Verbindung der Formel I zu erhalten, oder Aktivieren einer Phosphatgruppe der Uridinnukleotidverbindung mit einem Aktivierungsmittel von einer der Formeln IIIa-c; und Reagieren mit einer geeigneten Verbindung der Formel II b-d, um eine Verbindung der Formel I zu erhalten; und
c) Reinigen der Verbindung der Formel I und der pharmazeutisch annehmbaren Salze davon durch Ionenaustauschchromatographie;
wobei
die Formel I 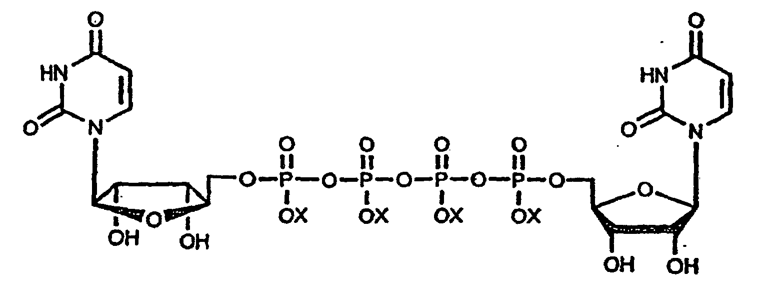 worin X aus der Gruppe: Na, NH₄ und H ausgewählt ist, vorausgesetzt, dass die ganzen X-Gruppen nicht H sind; 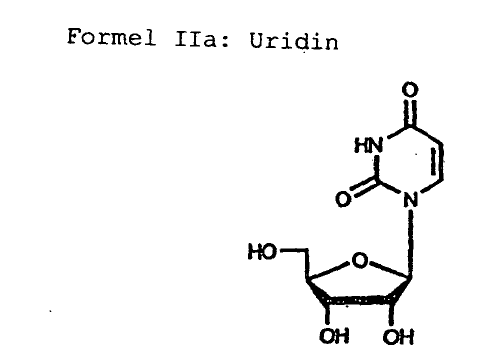 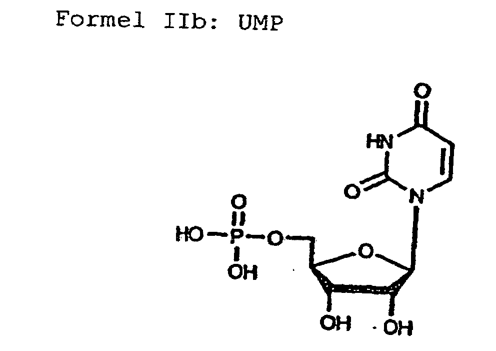 und Salze davon; 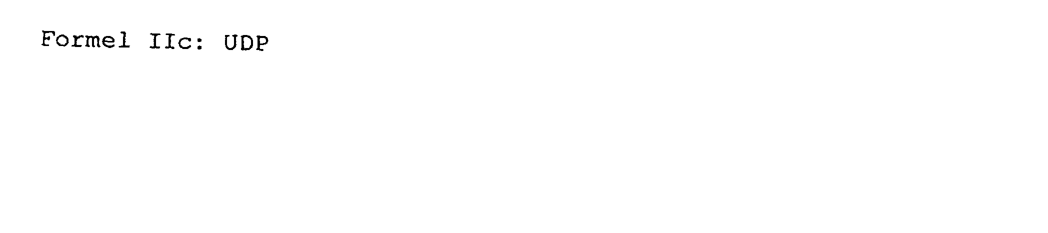 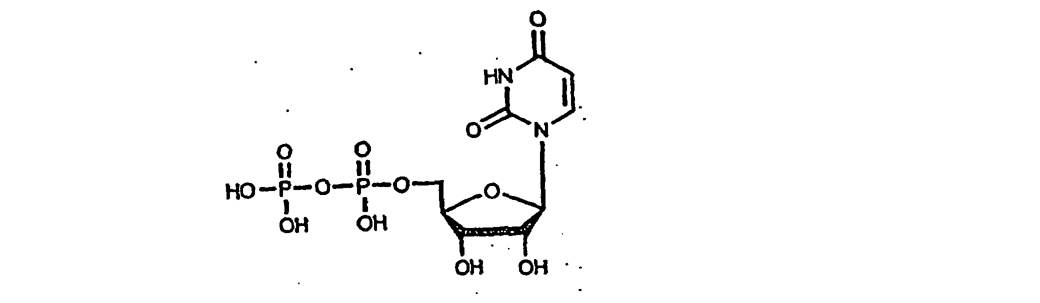 und Salze davon; 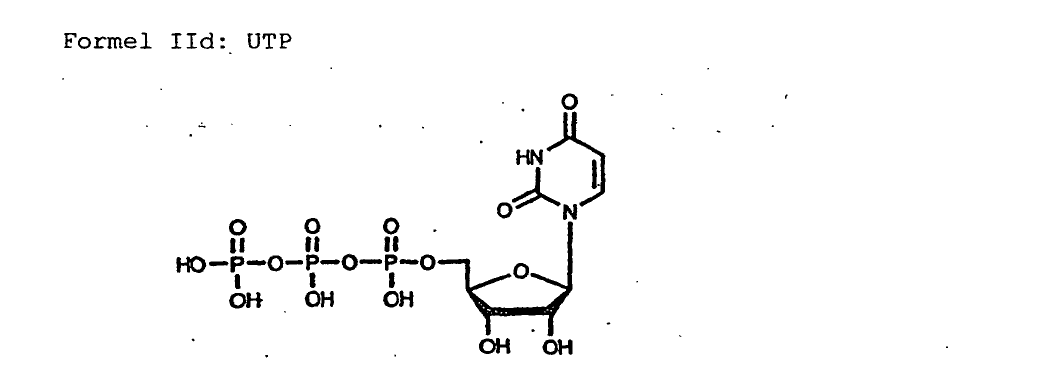 und Salze davon; 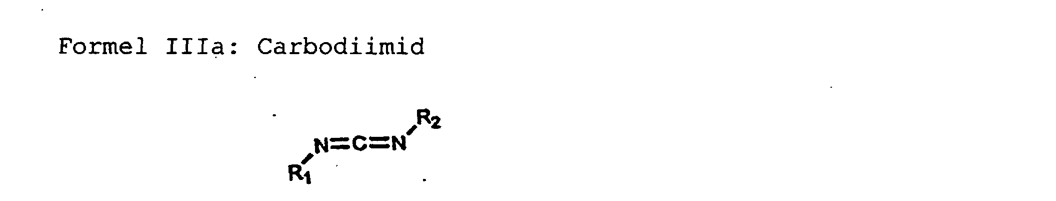 worin R₁ und R₂ unabhängig voneinander aus der Gruppe: C₁-C₈-Alkyl, Cycloalkyl und Aryl ausgewählt sind, wobei das Alkyl, Cycloalkyl oder Aryl wahlweise mit Hydroxyl oder Aminogruppen substituiert ist; 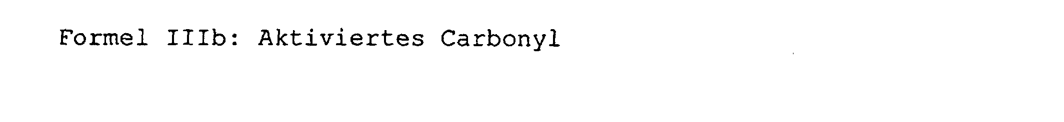 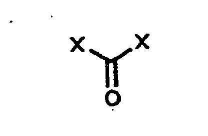 worin X unabhängig aus der Gruppe: Imidazol, Tetrazol und Halogen ausgewählt ist; 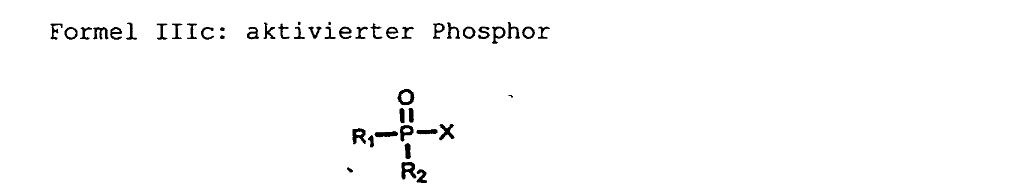 worin X Halogen ist und R₁ und R₂ jeweils unabhängig voneinander aus der Gruppe: C₁-C₈-Alkyl, Cycloalkyl, C₁-C₈-Alkoxy, Cycloalkoxy, Aryl, Aryloxy und Halogen ausgewählt sind, worin das Alkyl, Cycloalkyl, Alkoxy, Cycloalkoxy, Aryl oder Aryloxy wahlweise mit Hydroxyl, Amino oder Halogengruppen substituiert ist; 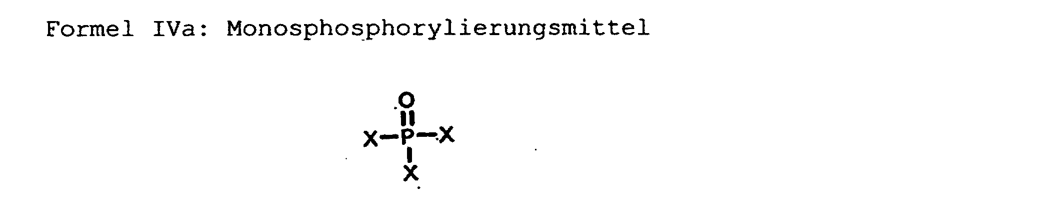 worin X Halogen ist; 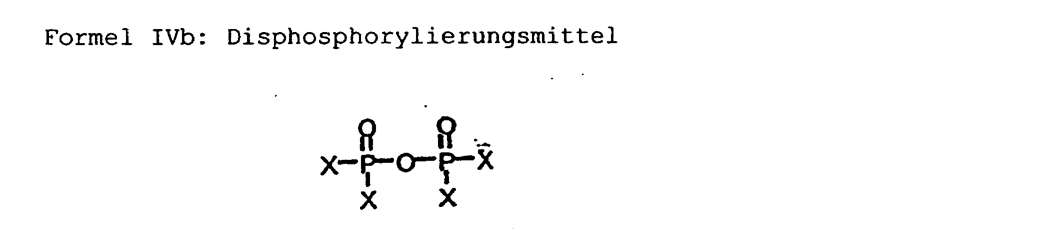 worin X aus der Gruppe: Sauerstoff, Hydroxy, Halogen und Salzen davon ausgewählt ist.

4. Verfahren nach Anspruch 3, wobei die Verbindungen der Formel IIIa aus der Gruppe: Dicyclohexylcarbodiimid und 1-(3-Dimethylaminopropyl)-3-ethylcarbodiimidhydrochlorid ausgewählt sind.

5. Verfahren nach Anspruch 3, wobei die Verbindungen der Formel IIIb aus der Gruppe: Carbonyldiimidazol und Carbonylditriazol ausgewählt sind.

6. Verfahren nach Anspruch 3, wobei die Verbindung der Formel IIIc aus der Gruppe: Diphenylphosphorchloridat, Phenylphosphordichloridat, Phenylphosphonsäuredichlorid und Diphenylphosphinsäurechlorid ausgewählt ist.

7. Verfahren nach Anspruch 3, wobei die Verbindung der Formel IVa Phosphoroxychlorid ist.

8. Verfahren nach Anspruch 3, wobei die Verbindung der Formel IVb aus der Gruppe: Pyrophosphorylchlorid und Pyrophosphat ausgewählt ist.

9. Verfahren nach Anspruch 3, umfassend das Auflösen einer Verbindung der Formel IIc, wie im Anspruch 3 beschrieben, in einem polaren, aprotischen organischen Lösungsmittel und einem hydrophoben Amin, das Behandeln mit einem Aktivierungsmittel von einer der Formeln IIIa-c, wie im Anspruch 3 beschrieben, und die nachfolgende Reinigung durch Chromatographie.

10. Verfahren zur Herstellung von Verbindungen der Formel I: 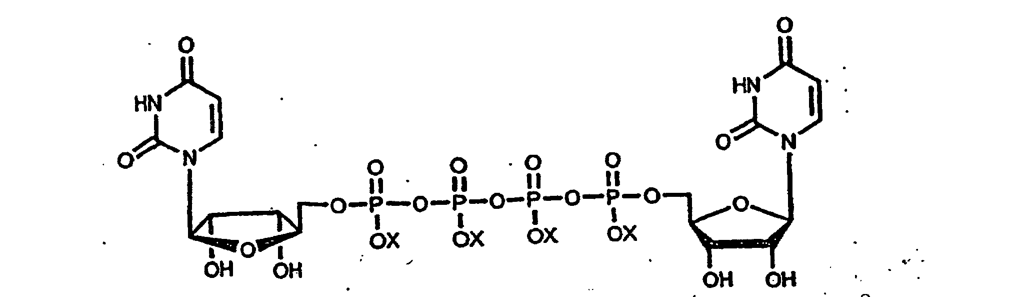 worin:
X aus der Gruppe: Na, NH₄ und H ausgewählt ist, vorausgesetzt, dass die ganzen X-Gruppen nicht H sind;
und pharmazeutisch annehmbaren Salzen davon, wobei das Verfahren das Auflösen einer Verbindung der Formel IIb, wie im Anspruch 3 beschrieben, in einem polaren, aprotischen organischen Lösungsmittel und einem hydrophoben Amin, das Behandeln mit einem Aktivierungsmittel aus den Formeln IIIa-c, wie im Anspruch 3 beschrieben, die nachfolgende Reaktion mit einer Verbindung der Formel IId, wie im Anspruch 3 beschrieben, und die anschließende Reinigung durch Chromatographie umfasst.

11. Verfahren nach Anspruch 3, umfassend das Auflösen einer Verbindung der Formel IId, wie im Anspruch 3 beschrieben, in einem polaren, aprotischen organischen Lösungsmittel und einem hydrophoben Amin, das Behandeln mit einer equimolaren Menge an Aktivierungsmittel aus einer der Formeln IIIa-c, wie im Anspruch 3 beschrieben, die nachfolgende Reaktion mit einer Verbindung der Formel IIb, wie im Anspruch 3 beschrieben, und die anschließende Reinigung durch Chromatographie.

12. Verfahren nach Anspruch 11, wobei ein Überschuss an Aktivierungsmittel verwendet wird.

13. Verfahren nach Anspruch 3, umfassend das Auflösen einer Verbindung der Formel IIb, wie im Anspruch 3 beschrieben, in einem polaren, aprotischen organischen Lösungsmittel und einem hydrophoben Amin, das Behandeln der Verbindung mit einem Aktivierungsmittel aus einer der Formeln IIIa-c, wie im Anspruch 3 beschrieben, die nachfolgende Reaktion mit einer geeigneten Verbindung der Formel IVb, wie im Anspruch 3 beschrieben, und die anschließende Reinigung durch Chromatographie.

14. Verfahren zur Herstellung von Verbindungen der Formel I: 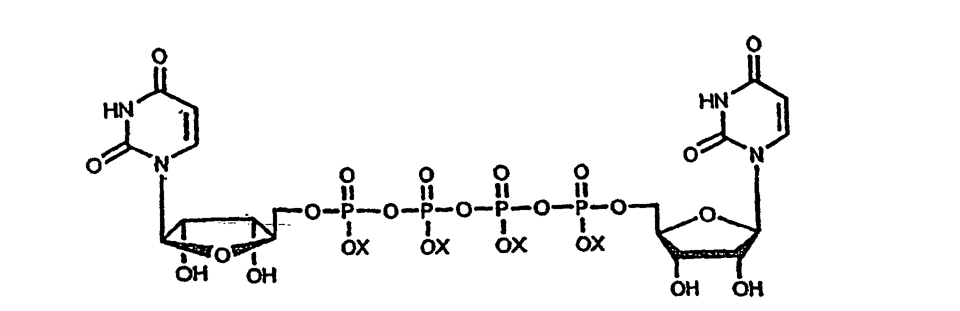 worin:
X aus der Gruppe: Na, NH₄ und H ausgewählt ist, vorausgesetzt, dass die ganzen X-Gruppen nicht H sind;
und pharmazeutisch annehmbaren Salzen davon, wobei das Verfahren das Auflösen einer Verbindung der Formel IIa, wie im Anspruch 3 beschrieben, in einem polaren, aprotischen organischen Lösungsmittel und einem hydrophoben Amin, das Behandeln mit Phosphorylierungsmitteln aus den Formeln IVa-b, wie im Anspruch 3 beschrieben, und die anschließende Reinigung durch Chromatographie umfasst.

15. Verfahren nach Anspruch 3, umfassend das Auflösen einer Verbindung der Formel IIb, wie im Anspruch 3 beschrieben, in einem polaren, aprotischen organischen Lösungsmittel und einem hydrophoben Amin, das Behandeln mit einem Phosphorylierungsmittel aus der Formel IVb, wie im Anspruch 3 beschrieben, und die anschließende Reinigung durch Chromatographie.

16. Verwendung einer Verbindung zur Herstellung eines Medikaments zum Behandeln eines Zustands, der aus der Gruppe chronische pulmonale Erkrankungen bei einem Säuger, Nasolakrimalgangobstruktion bei einem Säuger, Netzhautablösung bei einem Säuger, erleichterte Sputumeinleitung bei einem Säuger, erleichterte Expektoration bei einem Säuger und weibliche Unfruchtbarkeit oder Vaginalreizung aufgrund von vaginaler Trockenheit ausgewählt ist, wobei die Verbindung die Formel 1 aufweist: 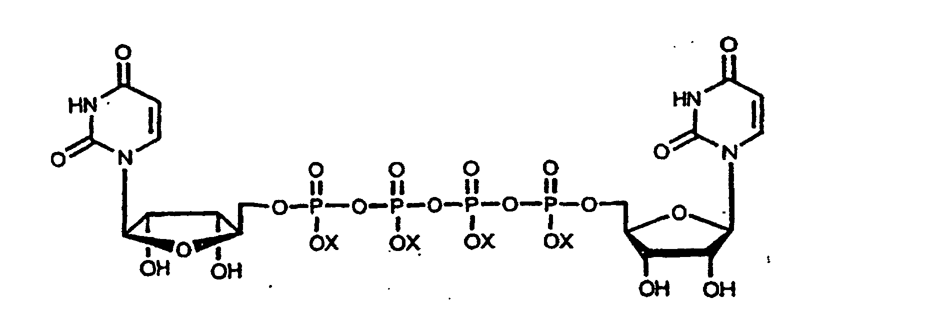 worin:
X aus der Gruppe: Na, NH₄ und H ausgewählt ist, vorausgesetzt, dass die ganzen X-Gruppen nicht H sind.

17. Pharmazeutische Zusammensetzung, umfassend P¹,P⁴-Di(uridin-5')-tetraphosphat, Tetranatriumsalz zusammen mit einem pharmazeutisch annehmbaren Träger dafür.

18. Pharmazeutische Zusammensetzung nach Anspruch 17, wobei die pharmazeutische Zusammensetzung steril ist.

19. Pharmazeutische Zusammensetzung nach Anspruch 17, wobei die pharmazeutische Zusammensetzung in der Form eines Gels, einer wässrigen Suspension, eines Granulats oder eines Pulvers vorliegt.

## Revendications

1. Sel tétrasodique de P¹,P⁴-di(uridine 5')-tétraphosphate.

2. Composition comprenant au moins 92% de sel de tétra-ammonium de P¹,P⁴-di(uridine 5')-tétraphosphate pur.

3. Procédé pour la synthèse de composés de formule I, et de leurs sels pharmaceutiquement acceptables, ledit procédé comprenant :
a) la dissolution d'uridine ou d'un composé nucléotidique d'uridine de formules IIa-d dans un solvant organique aprotique polaire et une amine hydrophobe ;
b) la phosphorylation avec un agent de phosphorylation répondant à l'une des formules IVa-b, ce qui donne un composé de formule I, ou l'activation d'un groupe phosphate et dudit composé nucléotique d'uridine avec un agent activateur répondant à l'une des formules IIIa-c ; et la réaction avec un composé convenable de formule IIb-d, ce qui donne un composé de formule I ;
c) la purification par chromatographie d'échange d'ions dudit composé de formule I et de ses sels pharmaceutiquement acceptables ;
où 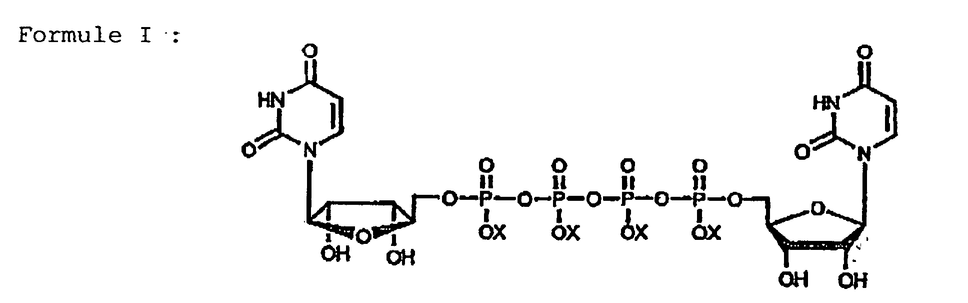 dans laquelle X est choisi dans le groupe consistant en :
Na, NH₄ et H, sous réserve que tous les groupes X ne représentent pas H ; 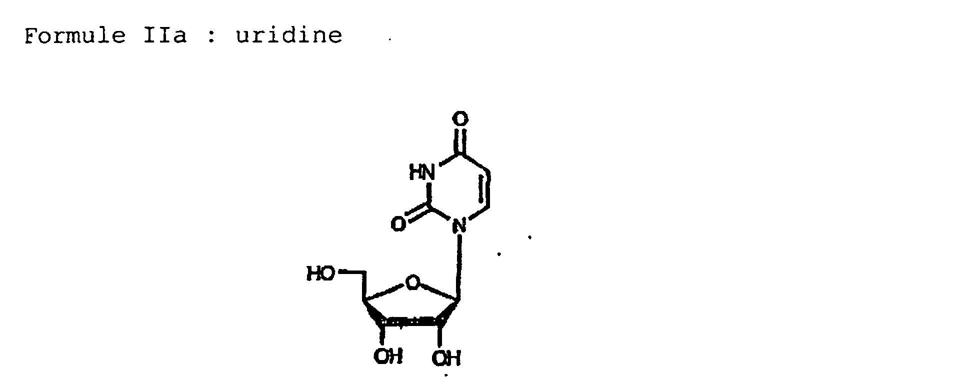 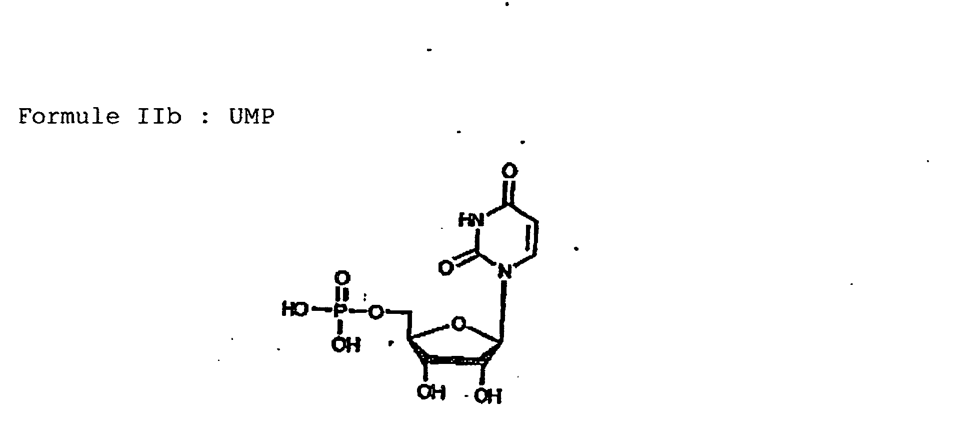
et ses sels ; 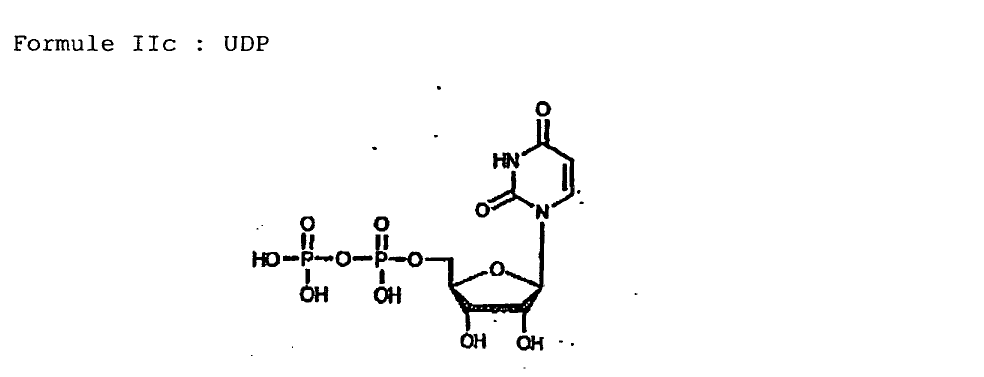 et ses sels ; 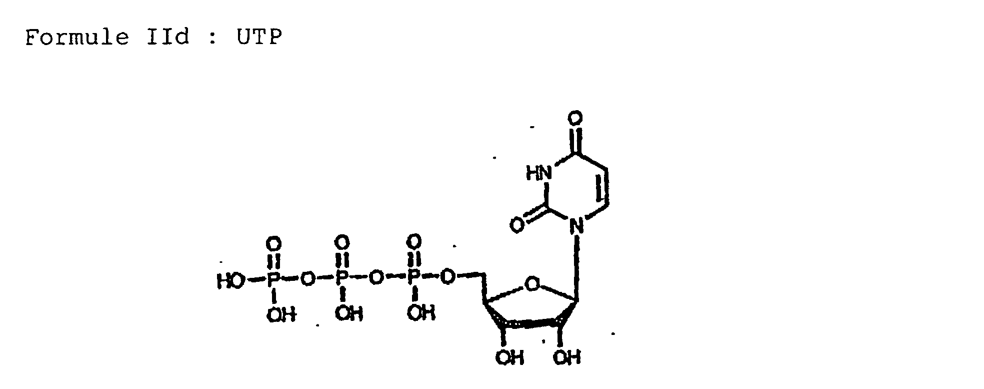 et ses sels ; 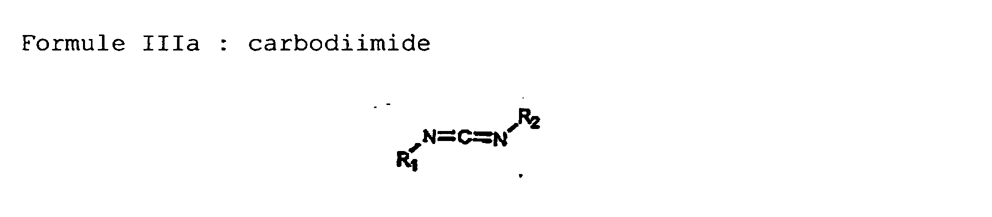 dans laquelle R₁ et R₂ sont choisis indépendamment dans le groupe consistant en des groupes : alkyle en C₁ à C₈, cycloalkyle et aryle, un tel groupe alkyle, cycloalkyle ou aryle étant facultativement substitué avec des groupes hydroxyle ou amino ; 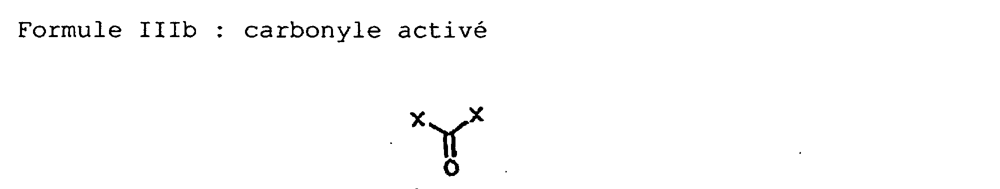 dans laquelle X est choisi indépendamment dans le groupe consistant en les groupes : imidazole, tétrazole et halogéno ; Formule IIIc : phosphore activé 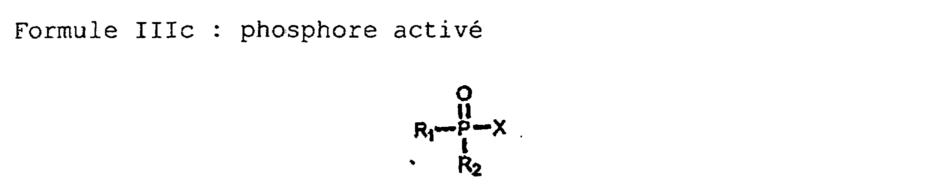 dans laquelle X représente un atome d'halogène et R₁ et R₂ sont choisis chacun indépendamment dans le groupe consistant en des groupes : alkyle en C₁ à C₈, cycloalkyle, alkoxy en C₁ à C₈, cycloalkoxy, aryle, aryloxy et halogéno, un tel groupe alkyle, cycloalkyle, alkoxy, cycloalkoxy, aryle, aryloxy étant facultativement substitué avec des groupes hydroxyle, amino ou halogéno ;
Formule IVa : agents de monophosphorylation 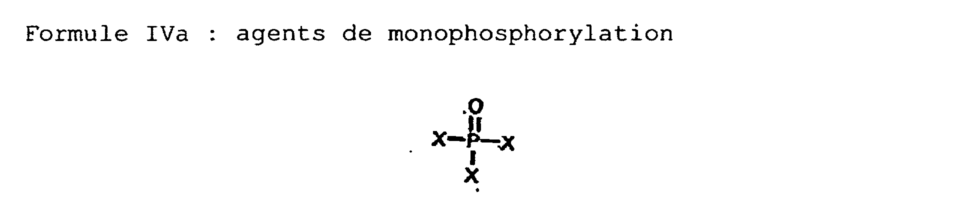 dans laquelle X représente un atome d'halogène ; 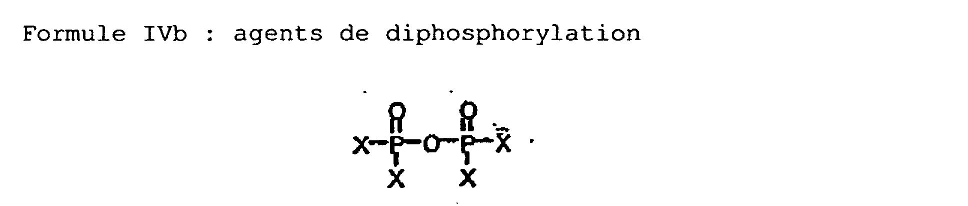 dans laquelle X est choisi dans le groupe consistant en :
un atome d'oxygène, un groupe hydroxy, un atome d'halogène, et ses sels.

4. Procédé suivant la revendication 3, dans lequel les composés de formule III sont choisis dans le groupe consistant en : le dicyclohexylcarbodiimide et le chlorhydrate de 1-(3-diméthylaminopropyl)-3-éthylcarbodiimide.

5. Procédé suivant la revendication 3, dans lequel les composés de formule IIIb sont choisis dans le groupe consistant en : le carbonyldiimidazole et le carbonylditriazole.

6. Procédé suivant la revendication 3, dans lequel le composé de formule IIIc est choisi dans le groupe consistant en : le phosphorochlorydate de diphényle, le phosphorodichloridate de phényl, le dichlorure phénylphosphonique et le chlorure diphénylphosphinique.

7. Procédé suivant la revendication 3, dans lequel le composé de formule IVa est l'oxychlorure de phosphore.

8. Procédé suivant la revendication 3, dans lequel le composé de formule IVb est choisi dans le groupe consistant en : le chlorure de pyrophosphoryle et un pyrophosphate.

9. Procédé suivant la revendication 3, comprenant la dissolution d'un composé de formule IIc répondant à la description figurant dans la revendication 3 dans un solvant organique aprotique polaire et une amine hydrophobe, le traitement avec un agent activateur répondant à l'une des formules IIIa à c suivant la description figurant dans la revendication 3, et ensuite une purification par chromatographie.

10. Procédé pour la préparation de composés de formule I : 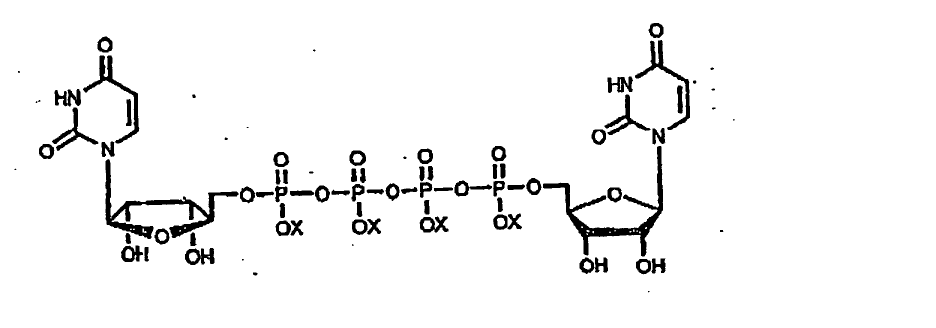 dans laquelle :
X est choisi dans le groupe consistant en : Na, NH₄ et H, sous réserve que tous les groupes X ne représentent pas H ; et de leurs sels pharmaceutiquement acceptables, ledit procédé comprenant la dissolution d'un composé de formule IIb répondant à la description figurant dans la revendication 3 dans un solvant organique aprotique polaire et une amine hydrophobe, le traitement avec un agent activateur répondant aux formules IIIa-c suivant la description figurant dans la revendication 3, puis une réaction avec un composé de formule IId suivant la description figurant dans la revendication 3, et ensuite une purification par chromatographie.

11. Procédé suivant la revendication 3, comprenant la dissolution d'un composé de formule IId suivant la description figurant dans la revendication 3 dans un solvant organique aprotique polaire et une amine hydrophobe, le traitement avec une quantité équimolaire d'un agent activateur répondant à l'une des formules IIIa-c suivant la description figurant dans la revendication 3, puis une réaction avec un composé de formule IIb dans la description figurant dans la revendication 3, et ensuite une purification par chromatographie.

12. Procédé suivant la revendication 11, dans lequel un excès d'agent activateur est utilisé.

13. Procédé suivant la revendication 3, comprenant la dissolution d'un composé de formule IIb suivant la description figurant dans la revendication 3 dans un solvant organique aprotique polaire et une amine hydrophobe, le traitement dudit composé avec un agent activateur répondant à l'une des formules IIIa-c suivant la description figurant dans la revendication 3, puis une réaction avec un composé de formule IVb suivant la description figurant dans la revendication 3, et ensuite une purification par chromatographie.

14. Procédé pour la préparation de composés de formule I : 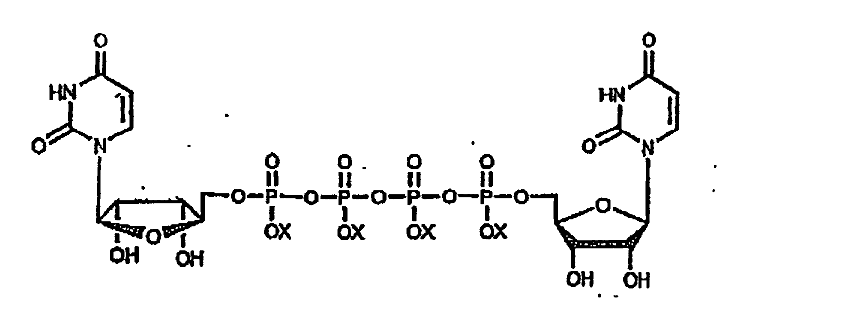 dans laquelle :
X est choisi dans le groupe consistant en : Na, NH₄ et H, sous réserve que tous les groupes X ne représentent pas H ;
et de leurs sels pharmaceutiquement acceptables, ledit procédé comprenant la dissolution d'un composé de formule IIa suivant la description figurant dans la revendication 3 dans un solvant organique aprotique polaire et une amine hydrophobe, le traitement avec des agents de phosphorylation répondant aux formules IVa-b suivant la description figurant dans la revendication 3, et ensuite une purification par chromatographie.

15. Procédé suivant la revendication 3, comprenant la dissolution d'un composé de formule IIb suivant la description figurant dans la revendication 3 dans un solvant organique aprotique polaire et une amine hydrophobe, le traitement avec un agent de phosphorylation de formule IV-b suivant la description figurant dans la revendication 3, et ensuite une purification par chromatographie.

16. Utilisation d'un composé pour la production d'un médicament destiné au traitement d'une affection choisi dans le groupe consistant en des maladies pulmonaires chroniques chez un mammifère, une obstruction du conduit lacrymonasal chez un mammifère, un décollement de la rétine chez un mammifère, l'amélioration de l'induction des expectorations chez un mammifère, l'amélioration de l'expectoration chez un mammifère et de la stérilité féminine ou de l'irritation vaginale due à une sécheresse vaginale, dans laquelle ledit composé répond à la formule I : 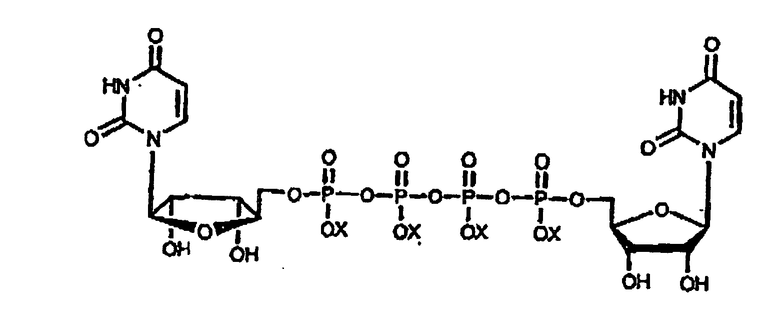 dans laquelle :
X est choisi dans le groupe consistant en : Na, NH₄ et H, sous réserve que tous les groupes X ne représentent pas H.

17. Composition pharmaceutique comprenant du sel tétrasodique de P¹,P⁴-di(uridine 5')-tétraphosphate conjointement avec un support pharmaceutiquement acceptable à cette fin.

18. Composition pharmaceutique suivant la revendication 17, ladite composition pharmaceutique étant stérile.

19. Composition pharmaceutique suivant la revendication 17 , ladite composition pharmaceutique étant sous forme d'un gel, d'une suspension aqueuse, de granules ou d'une poudre.
